(19) Europäisches Patentamt
European Patent Office
Office européen des brevets

(11) EP 4 351 590 B1

(12) EUROPEAN PATENT SPECIFICATION

(45) Date of publication and mention of the grant of the patent:
**10.12.2025 Bulletin 2025/50**

(21) Application number: **22820644.7**

(22) Date of filing: **13.06.2022**

(51) International Patent Classification (IPC):
***C07H 15/26*** (2006.01) ***A61K 31/7052*** (2006.01)
***A61P 37/00*** (2006.01) ***A61P 19/02*** (2006.01)
***A61P 29/00*** (2006.01) ***A61P 17/06*** (2006.01)
***C07H 1/00*** (2006.01)

(52) Cooperative Patent Classification (CPC):
**A61P 17/06; A61K 31/7052; A61P 19/02; A61P 29/00; A61P 37/00; C07H 1/00; C07H 15/26**

(86) International application number:
**PCT/PL2022/050037**

(87) International publication number:
**WO 2022/260546 (15.12.2022 Gazette 2022/50)**

# (54) A GLUCOSE-METHOTREXATE CONJUGATE FOR USE IN PREVENTING OR TREATING AUTOIMMUNE DISEASES

GLUCOSE-METHOTREXAT-KONJUGAT ZUR VERWENDUNG BEI DER VORBEUGUNG ODER BEHANDLUNG VON AUTOIMMUNERKRANKUNGEN

CONJUGUÉ GLUCOSE-MÉTHOTREXATE DESTINÉ À ÊTRE UTILISÉ DANS LA PRÉVENTION OU LE TRAITEMENT DE MALADIES AUTO-IMMUNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2021 PL 43813521**

(43) Date of publication of application:
**17.04.2024 Bulletin 2024/16**

(73) Proprietor: **Uniwersytet Medyczny Im. Piastów Slaskich We Wroclawiu**
**50-367 Wroclaw (PL)**

(72) Inventors:
- **AGRAWAL, Siddarth**
  **55-010 Zerniki Wroclawskie (PL)**
- **WOZNIAK, Marta**
  **50-067 Wroclaw (PL)**
- **MAKUCH, Sebastian**
  **34-424 Skrzypne (PL)**
- **SZEJA, Wieslaw**
  **44-100 Gliwice (PL)**
- **PASTUCH-GAWOLEK, Gabriela**
  **44-121 Gliwice (PL)**
- **KRAWCZYK, Monika**
  **32-300 Olkusz (PL)**
- **WISNIEWSKI, Jerzy**
  **51-423 Wroclaw (PL)**
- **GAMIAN, Andrzej**
  **51-644 Wroclaw (PL)**
- **ZIOLKOWSKI, Piotr**
  **52-430 Wroclaw (PL)**
- **MAZUR, Grzegorz**
  **53-204 Wroclaw (PL)**

(74) Representative: **Witek, Rafal**
**WTS Patent Attorneys**
**Witek, Sniezko & Partners**
**Ul. Rudolfa Weigla 12**
**53-114 Wroclaw (PL)**

(56) References cited:
**EP-A1- 1 739 097     PL-A1- 426 731**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ROSARIO PIGNATELLO ET AL.: "Lipophilic methotrexate conjugates with glucose-lipoamino acid moieties: Synthesis and in vitro antitumor activity", DRUG DEVELOPMENT RESEARCH, vol. 52, no. 3, March 2001 (2001-03-01), pages 454 - 461, XP055312801, Retrieved from the Internet <URL:https://doi.org/10.1002/ddr.1147> DOI: 10.1002/ddr.1147

## Description

Disease-modifying antirheumatic drugs (DMARDs) are commonly used to treat rheumatoid arthritis and psoriasis. Methotrexate (MTX) is still used today as a first-line or second-line drug in the treatment of patients with rheumatoid arthritis (RA) or psoriasis, although the beneficial effects of treatment are achieved only in 1/3 of patients. The main factor limiting the effectiveness of the treatment is the toxicity caused by the systemic effects of MTX. It is estimated that approximately 30% of patients discontinue therapy due to the high toxicity of the compound in the first year of treatment. Due to this problem, new, properly designed compounds are needed to solve the problem of the lack of therapeutic methods characterized by high efficacy and low toxicity.

In order to obtain high efficacy and reduce MTX toxicity, a compound was designed in which glucose is linked to the therapeutic compound via a linker. Glucose is a preferred bioenergetic and synthetic substrate for rapidly proliferating inflammatory cells. The hypothesis is based on the observation of specific biochemical and pathophysiological characteristics of cells in autoimmune diseases, such as increased glucose demand of hyperproliferating cells or overexpression of GLUT family transporters [Zhang, Z. et al. Differential glucose requirement in skin homeostasis and injury identifies a therapeutic target for psoriasis. Nature Medicine. 24, 617-627 (2018)].

It is assumed that the conjugation of a commonly known cytostatics with glucose via a cleavable linker may show increased bioavailability, selectivity, and reduced toxicity, as well as initiate an innovative method of treating autoimmune diseases [Makuch, S. et al. Glycoconjugation as a Promising Treatment Strategy for Psoriasis. Journal of Pharmacology and Experimental Therapeutics. May 2020, 373 (2) 204-212].

EP 1 739 097 A1 (DENKI KAGAKU KOGYO KK; CHUGAI PHARMACEUTICAL CO LTD) 3 January 2007 (2007-01-03) discloses compounds for the treatment of rheumatoid arthritis in the form of a methotrexate-glycoconjugate.

Side effects

MTX is currently available as an injectable solution or tablet for oral administration. While it is by far one of the most effective drugs in the treatment of psoriasis and rheumatoid arthritis, pharmacological limitations limit the use of MTX and result in an insufficient clinical response. The bioavailability of MTX at low doses is almost complete but drops to only 10-20% at higher doses. MTX is rapidly eliminated from the circulation via the kidneys (5-8 hours plasma half-life), resulting in low drug concentrations in target tissues, e.g., the epidermis and inflamed joints. The main reason for discontinuing MTX treatment is not lack of efficacy but high toxicity. The most common symptoms are gastrointestinal toxicity, such as stomatitis, nausea, and abdominal discomfort. While the exact mechanisms of the toxicity are still unclear, some side effects are directly related to the effects of MTX on major metabolic pathways. 7-OH-MTX, the major metabolite of MTX, is sparingly soluble in water and may contribute to renal toxicity in high doses. The above side effects limit the use of MTX at higher doses.

To improve drug efficacy, it is necessary to develop targeted therapies that allow increased uptake of the active substance by inflammatory cells compared to normal cells. Much research has been done in recent years to overcome these limitations using new drug delivery systems. These systems provide many benefits, such as improved drug safety and efficacy, improved bioavailability, prolonged drug action in the target tissue, and improved stability of the therapeutic compounds (protection against chemical and enzymatic degradation).

The metabolic properties of inflammatory cells differ significantly from those of normal cells. These differences open the way to the development of new compounds with high affinities for affected cells. One promising strategy to address metabolic differences in inflammatory cells is glucose-drug conjugation. This approach takes advantage of the increased demand for glucose by fibroblast-like synoviocytes (RA) or a hyperproliferating epidermis (psoriasis) compared to normal tissue and the overexpression of glucose transporters (GLUT) in these affected tissues [Zhang, Z. et al. Differential glucose requirement in skin homeostasis and injury identifies a therapeutic target for psoriasis. Nature Medicine. 24, 617-627 (2018); Makuch, S. et al. Glycoconjugation as a Promising Treatment Strategy for Psoriasis. Journal of Pharmacology and Experimental Therapeutics. May 2020, 373 (2) 204-212; Garcia-Carbonell, R. et al. Critical Role of Glucose Metabolism in Rheumatoid Arthritis Fibroblast-like Synoviocytes. Arthritis & rheumatology (Hoboken, N.J.) vol. 68.7 (2016): 1614-26]. These conjugates are designed to be recognized by specific GLUT receptors and preferentially absorbed by affected cells.

The object of the invention is to provide a drug which is useful in the treatment and prevention of autoimmune diseases. In particular, it is desirable that this drug exhibit better specificity than methotrexate and also overcome drug resistance, and exhibit prolonged activity in the bloodstream.

The essence of the invention

Unexpectedly, the technical problem defined in this way has been solved in the present invention. The present invention relates to a glucose-methotrexate conjugate of the formula:

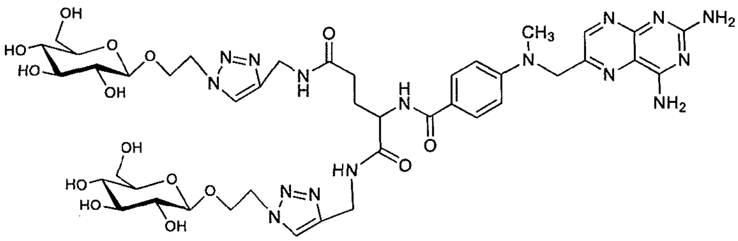

for use in the prevention or treatment of autoimmune diseases.

Preferably, the autoimmune disease is rheumatoid arthritis or psoriasis.

Detailed description of the invention

The subject of the invention is shown in an exemplary embodiment and in the attached figures, where:

Figure 1 shows the viability of TNF-a stimulated SW982 cells after treatment with 10 μM MTX and Glu-MTX for 48 h, MTT assay. * statistical significance between sample and control ($p < 0.05$).

Figure 2 shows the viability of TNF-a stimulated HaCaT cells after treatment with 10 μM MTX and Glu-MTX for 48 h, MTT assay. * statistical significance between sample and control ($p < 0.05$).

Figure 3 shows the viability of unstimulated HaCaT cells after treatment with 10 μM MTX and Glu-MTX for 48 h, MTT test. * statistical significance between sample and control ($p < 0.05$).

Figure 4 shows the levels of TNF-a, IL-17, and IL-23 after treatment with exogenous TNF-a and MTX or Glu-MTX (pg/ml). * statistical significance between sample and control ($p < 0.05$).

**Synthesis**

The synthesis of a methotrexate prodrug with a glycoconjugate structure has not been proposed so far. The designed prodrug contains D-glucose linked via a difunctional linker to methotrexate. The combination of a difunctional linker with sugar and methotrexate was designed so that when the prodrug was introduced into a cell overexpressing GLUT transporters, MTX was released through glycosidic and amide bond hydrolysis catalyzed by glycosylhydrolase and peptidase hydrolytic enzymes. The sugar substrate is tetra-O-acetyl-3-azidopropyl-β-D-glucoside. An intermediate substrate, a methotrexate derivative, is a diamide, a reaction product of propargylamine with carboxyl groups. When planning the glycoconjugate synthesis, a number of assumptions were made that determine the effectiveness of the method. Thus, due to the observed isomerization of the glutamic acid residue in carboxyl functionalization reactions, this amide bond formation step was planned to be a carbodiimide catalyzed condensation reaction in the presence of 1-hydroxybenztriazole. Linkage of the substrates was planned to be a 1,3-dipolar cycloaddition of the azide to a terminal triple bond to form a triazine. In a variant developed by Sharples, this is a commonly used method of preparing prodrugs. The final stage of the synthesis is the deacylation of the sugar unit. The conventional method is an alcoholysis reaction catalyzed by sodium methoxide under the conditions described by Zemplen. Under the selected conditions, sugar deprotection did not lead to the lysis of the amide bond. The product was isolated by column chromatography.

The detailed synthesis of the prodrug is shown below.

**Example 1.**

**Chemical synthesis**

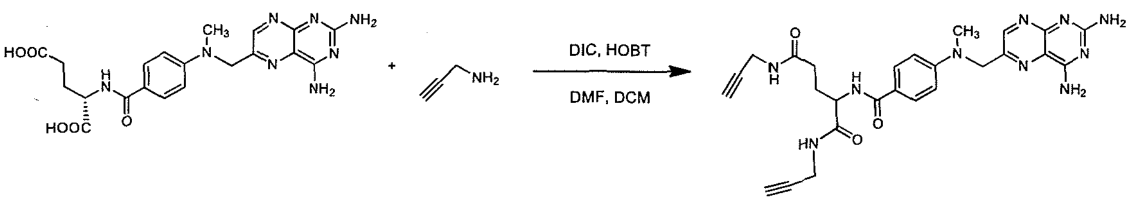

The method of making a methotrexate prodrug includes:

1) amide synthesis - dissolving MTX in dry DMF, heating at a temperature of 20 °C to 30 °C, to dissolve MTX, adding methylene chloride to obtain a saturated MTX solution, then cooling to 0 °C, adding N,N-diisopropylcarbodiimide (DIC), 1-hydroxybenzotriazole (HOBT) and propargylamine. The mole ratio of MTX/DIC/HOBT/amine is 1/1.2/0.5/2.2. The amidation reaction was carried out at room temperature while stirring the contents of the vessel for 10 days. After the concentration of the reaction mixture, the product was then isolated by column chromatography on silica gel.
2) glycoconjugate synthesis by 1,3-dipolar cycloaddition: the separated, chromatographically pure diamide was dissolved in isopropyl alcohol/THF (1/1; v/v). 3-Azidopropyl tetra-O-acetyl-β-D-glucopyranoside was added to the resulting solution and stirred until a yellow solution was obtained. Thereafter, an aqueous solution of sodium ascorbate and an aqueous solution of copper (II) sulfate were added. The reaction was carried out at room temperature for 24 hours. After completion of the reaction (complete conversion of the diamide), the precipitate was filtered off, the filtrate was concentrated, and the product was isolated by column chromatography on silica gel.
3) methanolysis - deacylation, the obtained glycoconjugate was dissolved in methyl alcohol (5 ml), a solution of sodium methoxide in methanol (1 ml) was added, stirred at room temperature for 30 minutes, hydrogen cation exchanger was added to neutralize the solution, the cation exchanger was filtered off, washed with methanol, the filtrates were concentrated under reduced pressure to afford chromatographically pure methotrexate conjugate prodrug as yellow solid.

The described reactions were carried out by protecting the reaction vessels from light.

**Step I: Synthesis of the propargylamide derivative of methotrexate**

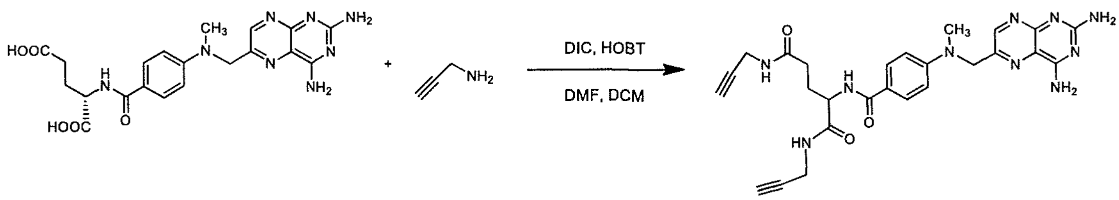


Methotrexate (500 mg, 1.10 mmol) was dissolved in anhydrous DMF (10 ml) and anhydrous DCM (2 ml). The flask was protected from light and placed in an ice/water bath. DIC (210 μL, 1.34 mmol) was added dropwise to the reaction mixture at 0 °C, and HOBT (77 mg, 0.57 mmol) was added, followed by propargylamine (155 μL, 2.42 mmol). The reaction was carried out at room temperature for 10 days. After quenching the reaction, the contents of the flask were concentrated under reduced pressure. The crude product was purified by column chromatography on silica gel (50:1 to 10:1 $CHCl_3$:$CH_3OH$ gradient elution) to give a yellow solid (407 mg, 70%; m.p.: 105 °C, $\alpha_D^{23} = 16$ (c = 1.0, DMSO)).

$^1$H NMR (400 MHz, DMSO-d6): δ 1.80-2.05 (m, 2H, $2xCH_{MTX}$), 2.07-2.24 (m, 2H, $CH_{2MTX}$), 3.06 (m, 1H, CH), 3.16 (m, 1H, CH), 3.22 (s, 3H, $CH_{3MTX}$), 3.80-3.85 (m, 4H, $2xCH_2$), 4.33 (m, 1H, $CH_{MTX}$), 4.80 (s, 2H, $CH_{2MTX}$), 6.78-6.85 (m, 4H, $2xH\text{-}PH_{MTX}$, $NH_{2MTX}$), 7.63 (bs, 1H, $NH_{MTX}$), 7.72-7.77 (m, 2H, $2xH\text{-}PH_{MTX}$), 7.85 (bs, 1H, $NH_{MTX}$), 8.09 (m, 1H, $NH_{MTX}$), 8.23-8.31 (m, 2H, 2xNH), 8.58 (s, 1H, $H\text{-}7_{MTX}$). $^{13}$C NMR (100 MHz, DMSO-d6): δ 27.36, 27.76, 27.94, 31.77, 38.87, 52.90, 54.82, 72.81, 72.83, 81.13, 81.17, 110.92, 110.99, 121.17, 121.46, 128.90, 128.97, 146.61, 149.06, 150.82, 152.54, 160.82, 162.64, 166.07, 171.38, 171.58.

HRMS (ESI-TOF): calculated for $C_{26}H_{28}N_{10}O_3$ ($[M + H]^+$): m/z 529.2424, found: m/z 529.2421 .

**Step II: Synthesis of a glycoconjugate, a derivative of methotrexate**

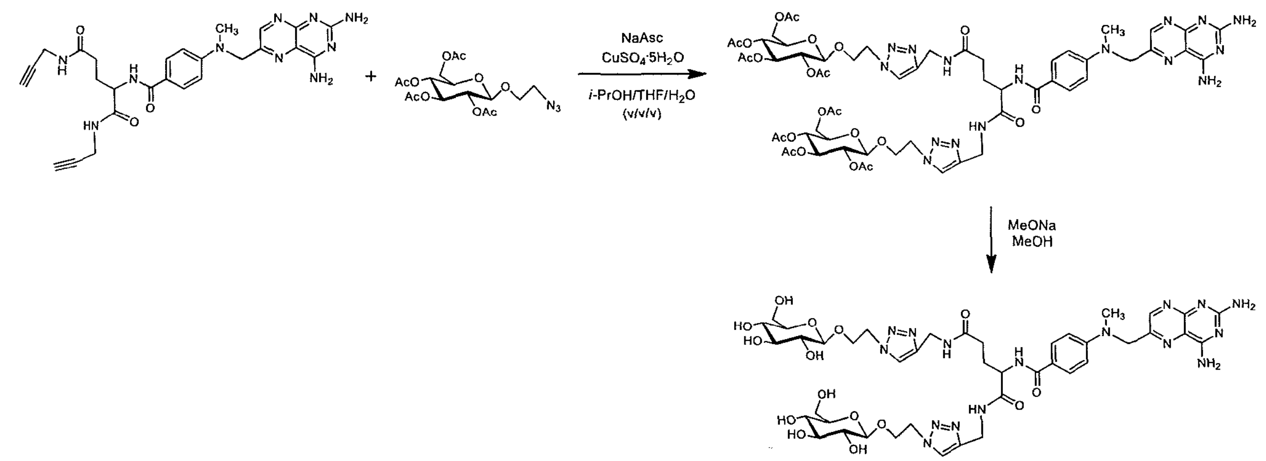


The propargylamide derivative of methotrexate (407 mg, 0.77 mmol) was obtained in the previous step, and sugar azide (643 mg, 1.54 mmol) was dissolved in a mixture of *i*-PrOH (6 ml) and THF (6 ml). Catalytic systems were prepared in two vials: sodium ascorbate (123 mg, 0.62 mmol) dissolved in $H_2O$ (3 ml) and $CuSO_4 \cdot 5H_2O$ (77 mg, 0.31 mmol) dissolved in $H_2O$ (3 ml), they were mixed together and added to the reaction mixture. The reaction was carried out at room temperature for 24 h. After completion of the reaction, the contents of the flask were concentrated under reduced pressure. The crude product was purified by silica gel column chromatography (20:1 to 5:1 $CHCl_3$:$CH_3OH$ gradient elution) to give a yellow solid (724 mg, 69%; m.p.: 114 °C, $\alpha_D^{23} = -6$ (c = 1.0, DMSO).

$^1$H NMR (400 MHz, DMSO-d6): δ 1.87-1.91 (m, 2H, 2x$CH_{MTX}$), 1.92, 1.98, 2.01, 2.02 (4s, 24H, 8x$CH_3CO$), 2.11-2.24 (m, 2H, $CH_{2MTX}$), 3.21 (s, 3H, $CH_{3MTX}$), 3.84-3.93 (m, 2H, $CH_2NH$), 3.97 (ddd, 2H, *J* = 2.4 Hz, *J* = 4.9 Hz, *J* = 9.8 Hz, 2xH-$5_{glu}$), 4.03 (dd, 2H, *J* = 2.4 Hz, *J* = 12.3 Hz, 2xH-$6a_{glu}$), 4.05-4.12 (m, 2H, $CH_2NH$), 4.17 (dd, 2H, *J* = 4.9 Hz, *J* = 12.3 Hz, 2xH-$6b_{glu}$), 4.22-4.32 (m, 4H, 2x$CH_2O$), 4.36 (m, 1H, $CH_{MTX}$), 4.42-4.54 (m, 4H, 2x$CH_2N$), 4.72 (dd-t, 2H, *J* = 8.2 Hz, J = 9.4 Hz 2xH-$2_{glu}$), 4.79 (s, 2H, **$CH_{2MTX}$),** 4.82 (d, 2H, *J* = 8.2 Hz, 2xH-$1_{glu}$), 4.89 (dd~t, 2H, *J* = 9.4 Hz, *J* = 9.4 Hz, 2xH-$3_{glu}$), 5.22 (dd~t, 2H, *J* = 9.4 Hz, *J* = 9.8 Hz, 2xH-$4_{glu}$), 6.65 (bs, 2H, $NH_{2MTX}$), 6.81 (m, 2H, 2xH-$PH_{MTX}$), 7.74 (m, 2H, $NH_{2MTX}$), 7.78 (m, 2H, 2xH-$PH_{MTX}$), 8.08 (m, 1H, $NH_{MTX}$), 8.26-8.38 (m, 2H, 2xNH), 8.56 (s, 2H, 2xH-$5_{triaz}$), 8.59 (s, 1H, H-$7_{MTX}$).

$^{13}$C NMR (100 MHz, DMSO-d6): δ 20.19, 20.22, 20.34, 20.47, 27.55, 31.91, 34.15, 38.89, 49.11, 53.08, 54.83, 61.62, 67.38, 68.08, 70.52, 70.64, 71.91, 99.13, 110.99, 121.21, 121.41, 122.98, 128.96, 144.67, 146.16, 149.10, 150.86, 154.71, 162.55, 162.66, 166.09, 168.95, 169.23, 169.48, 170.02, 171.62, 171.76.

HRMS (ESI-TOF): calculated for $C_{58}H_{74}N_{16}O_{23}$ ([M + H]$^+$): m/z 1363.5191, found: m/z 1363.5137.

A weighed aliquot of the obtained glycoconjugate (300 mg, 0.22 mmol) was dissolved in methanol (20 mL), and then a 1M solution of sodium methoxide in methanol (600 μL, 0.6 mmol) was added. The reaction was carried out at room temperature for 3 h. After completion of the reaction, the contents of the flask were neutralized with Amberlyst-15 ion exchange resin, which was then filtered, and the filtrate was concentrated under reduced pressure to give the deprotected glycoconjugate as a yellow solid (183 mg, 81%; m.p.: 107-108 °C, $\alpha_D^{23} = 42$ (c = 1.0, DMSO).

$^1$H NMR (400 MHz, DMSO-d6): δ 1.85-2.10 (m, 2H, 2x$CH_{MTX}$), 2.13-2.28 (m, 2H, $CH_{2MTX}$), 3.21 (s, 3H, $CH_{3MTX}$), 2.93-3.01 (m, 2H, 2xH-$2_{Glu}$), 3.03-3.08 (m, 2H, 2xH-$4_{Glu}$), 3.09-3.16 (m, 4H, 2xH-$3_{Glu}$, 2xH-$5_{Glu}$), 3.39-3.49 (m, 2H, 2xH-$6a_{Glu}$), 3.63-3.70 (m, 2H, 2xH-$6b_{Glu}$), 3.83-3.93 (m, 4H, $CH_2NH$), 4.01-4.10 (m, 4H, 2x$CH_2$), 4.19-4.24 (m, 2H, $CH_2$), 4.25-4.32 (m, 4H, 2xH-$1_{Glu}$, 2x$CH_2$), 4.38 (m, 1H, $CH_{MTX}$), 4.47-4.56 (m, 4H, 4xOH), 4.78 (s, 2H, $CH_{2MTX}$), 6.60 (bs, 2H, $NH_{2MTX}$), 6.81 (m, 2H, 2xH-$PH_{MTX}$), 7.66 (bs, 2H, $NH_{2MTX}$), 7.74 (m, 2H, 2xH-$PH_{MTX}$), 8.15 (m, 1H, $NH_{MTX}$), 8.32-8.44 (m, 2H, 2xNH), 8.47 (s, 2H, 2xH-$5_{triaz}$), 8.56 (s, 1H, H-$7_{MTX}$).

$^{13}$C NMR (100 MHz, DMSO-d6): δ 30.24, 31.99, 34.21, 34.31, 38.62, 48.31, 49.31, 51.01, 54.55, 60.77, 66.99, 69.71, 73.00, 76.35, 76.73, 102.66, 110.74, 111.70, 120.34, 121.54, 123.25, 128.76, 144.35, 145.70, 148.86, 150.88, 154.88, 162.39, 162.55, 165.58, 171.57, 172.61.

HRMS (ESI-TOF): calculated for $C_{42}H_{58}N_{16}O_{15}$ ([M + H]$^+$): m/z 1027.4346, found: m/z 1027.5416.

The results of the conducted research show that the developed molecule shows strong inhibitory effects on the growth of affected cells in a proven model of RA and psoriasis, similar to the *in vitro* activity of MTX. Moreover, the results show that the developed compound has a lower affinity for healthy cells than MTX. The results of *in vivo* tests show that the compound has a favorable safety profile without causing systemic toxicity even at doses significantly higher than MTX (equivalent doses).

**Example 2. Analysis of the biological activity of the conjugate according to the invention in rheumatoid arthritis (RA) and psoriasis cell models**

**Cytotoxicity analysis of Glu-MTX compared to free MTX using the MTT assay**

**Methodology - MTT test**

The developed cell models of rheumatoid arthritis (human synoviocytes, stimulated by tumor necrosis factor (TNF-a), SW982 cell line) [Khansai, M., Phitak, T., Klangjorhor, J. et al. Effects of sesamin on primary human synovial fibroblasts and SW982 cell line induced by tumor necrosis factor-alpha as a synovitis-like model. BMC Complement Altern Med 17, 532 (2017)] and psoriasis (human keratinocytes, stimulated by TNF-a, HaCaT cell line) [Wu S, Zhao M, Sun Y, Xie M, Le K, Xu M, Huang C. The potential of Diosgenin in treating psoriasis: Studies from HaCaT keratinocytes and imiquimod-induced murine model. Life Sci. 2020 Jan 15; 241: 117115].

SW982 and HaCaT cells were seeded in a 96-well plate at 10 x $10^3$ cells per well. The next day, cells were incubated with TNF-a at a dose of 10 ng/ml to induce inflammation. After 24h, cells were treated with MTX or Glu-MTX at 10 $\mu$M dose and incubated for 48h. After 48 hours of incubation, the medium was decanted, and 100 $\mu$l of a solution of yellow soluble 3-(4,5-dimethyl-2-thiazolyl)-2,5-diphenyl-2H-tetrazolium bromide (MTT) at a concentration of 0.5-1 mg/ml was added. After 4 hours of incubation, the MTT solution was decanted, and 50 $\mu$l/well of DMSO was added. After 3 hours, the optical density of the dissolved formazan was analyzed colorimetrically in a BioTek plate reader.

The concentration of DMSO in the tested samples did not exceed 0.2%.

Since living cells reduce MTT to formazan, the optical density (OD) is highest in control (C) wells which accounted for 100% of cell viability. Viability in wells incubated with compound (COMP) was calculated using the formula:

$$X = 100\% \times OD\ COMP/OD\ C$$

**MTT test results**

Cell viability studies by MTT assay showed that Glu-MTX is cytotoxic *in vitro.* Figures 1 and 2 show the results of a viability assessment study ($IC_{50}$) for TNF-$\alpha$-stimulated SW982 and HaCaT cell lines treated with methotrexate alone or a glucose-methotrexate (Glu-MTX) derivative. The cytotoxic effect of the glucose-methotrexate conjugate is similar to that of unmodified methotrexate. The test was also performed on an unstimulated HaCaT cell line, which is the counterpart of non-inflammatory human keratinocytes. The results show that in a non-inflammatory cell model, methotrexate induces much greater cytotoxicity in contrast to Glu-MTX, which exhibits high selectivity, affecting much less healthy cells. The study used: 10 $\mu$M Glu-MTX, 10 $\mu$M Methotrexate; incubation 48 hours; MTT analysis. The results reported are the mean value of 3 replicates.

**Assessment of the level of cytokines and inflammation of SW982 and HaCaT cells after treatment with Glu-MTX and free MTX using the MILLIPLEX test**

**Methodology - MILLIPLEX test**

Cells were seeded in 6-well plates at 350,000 cells/well and a volume of 1000 $\mu$l. The next day, cells were incubated with TNF-a at a dose of 10 ng/ml to induce inflammation. After 24h, cells were treated with MTX or Glu-MTX at 10 $\mu$M dose and incubated for 48h. After incubation with the compound, the supernatant was harvested and transferred to sterile Eppendorf tubes from cell culture, and the levels of IL-6, IL-8, and IL-1$\beta$ were measured with the MILLIPLEX MAP Human Cytokine assay according to the manufacturer's instructions.

**MILLIPLEX test results**

The study of the levels of TNF-a, IL-17, and IL-23 using the MILLIPLEX MAP Human Cytokine test in the RA (SW982 line) and psoriasis (HaCaT line) cell model showed that Glu-MTX significantly reduces the level of cytokines that are a key role in the development of inflammation in autoimmune diseases. Figure 4 shows the levels of the cytokines TNF-a, IL17, and IL-23 in the culture medium of the SW982 and HaCaT cell lines after stimulation with exogenous TNF-a (10 ng/ml) and treatment with 10 $\mu$M MTX or 10 $\mu$M Glu-MTX. The results reported are the mean value of 3 replicates.

## Claims

1. Methotrexate glucose conjugate of a formula:

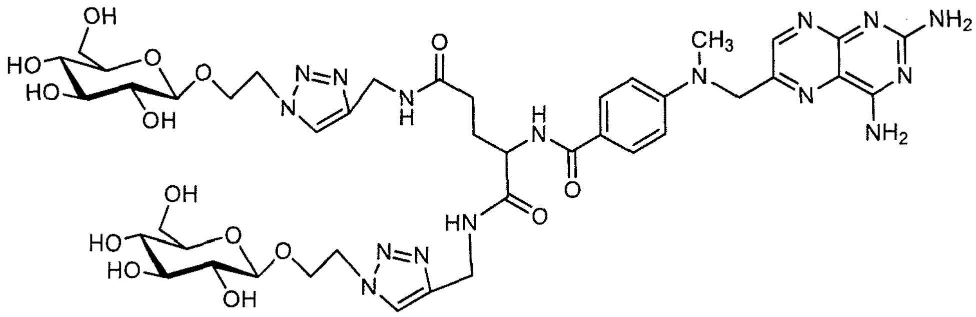

for use in the prevention or treatment of autoimmune diseases.

**2.** The conjugate for use according to claim 1, wherein the autoimmune disease is rheumatoid arthritis or psoriasis.

**Patentansprüche**

**1.** Glucose-Methotrexat-Konjugat einer Formel:

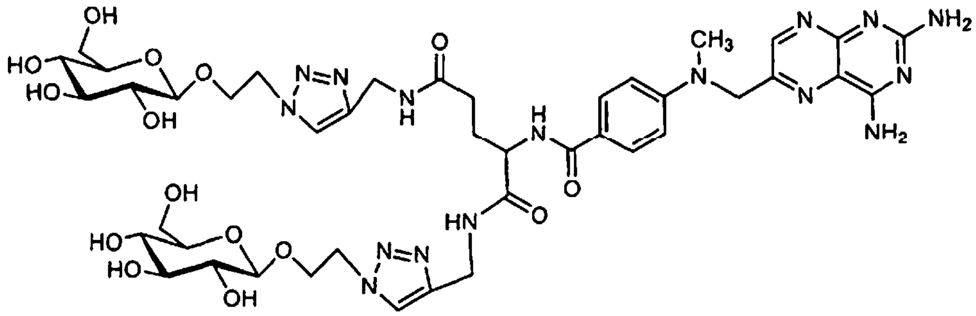

zur Verwendung bei der Vorbeugung oder Behandlung von Autoimmunerkrankungen.

**2.** Konjugat zur Verwendung nach Anspruch 1, wobei die Autoimmunkrankheit rheumatoide Arthritis oder Psoriasis ist.

**Revendications**

**1.** Conjugué de glucose méthotrexate d'une formule :

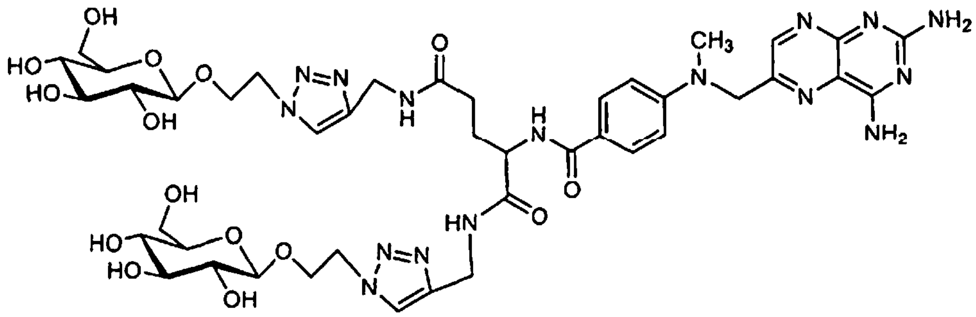

destiné à être utilisé dans la prévention ou le traitement de maladies auto-immunes.

**2.** Conjugué pour une utilisation selon la revendication 1, dans lequel la maladie auto-immune est la polyarthrite rhumatoïde ou le psoriasis.

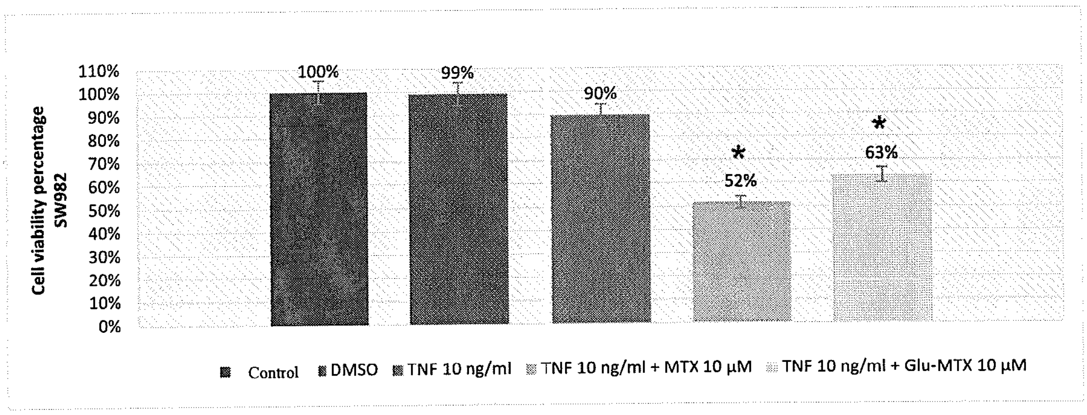


**Figure 1**

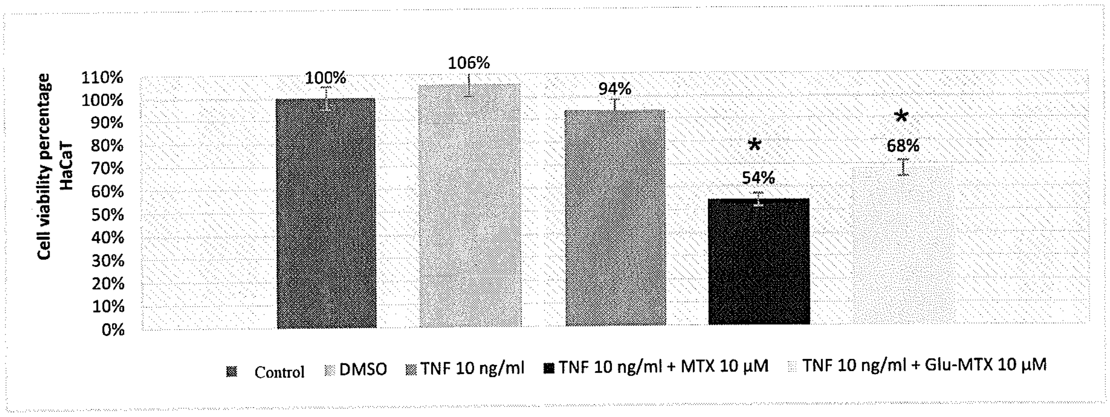


**Figure 2**

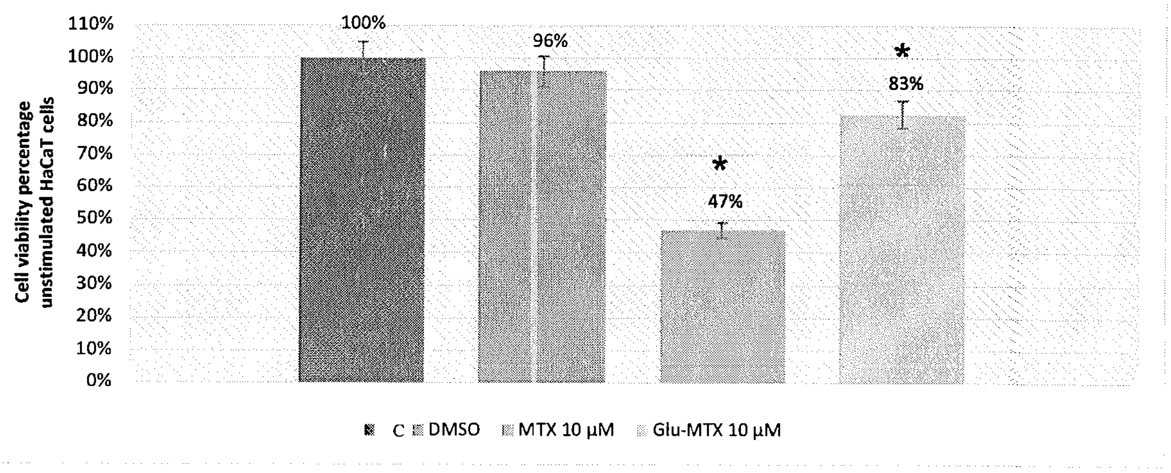


Figure 3

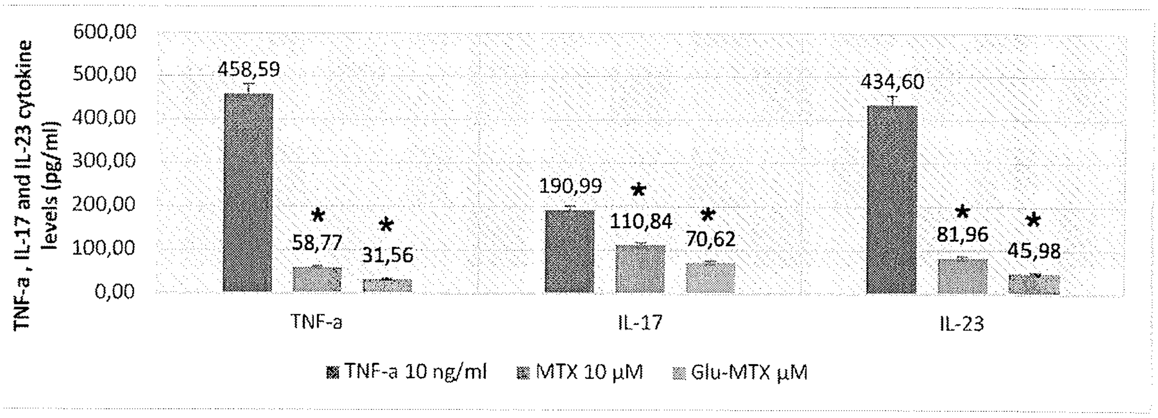


Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1739097 A1


### Non-patent literature cited in the description


- **ZHANG, Z. et al.** Differential glucose requirement in skin homeostasis and injury identifies a therapeutic target for psoriasis. *Nature Medicine*, 2018, vol. 24, 617-627
- **MAKUCH, S. et al.** Glycoconjugation as a Promising Treatment Strategy for Psoriasis. *Journal of Pharmacology and Experimental Therapeutics*, May 2020, vol. 373 (2), 204-212
- **GARCIA-CARBONELL, R. et al.** Critical Role of Glucose Metabolism in Rheumatoid Arthritis Fibroblast-like Synoviocytes. *Arthritis & rheumatology (Hoboken, N.J.)*, 2016, vol. 68 (7), 1614-26
- **KHANSAI, M.** ; **PHITAK, T.** ; **KLANGJORHOR, J. et al.** Effects of sesamin on primary human synovial fibroblasts and SW982 cell line induced by tumor necrosis factor-alpha as a synovitis-like model. *BMC Complement Altern Med*, 2017, vol. 17, 532
- **WU S** ; **ZHAO M** ; **SUN Y** ; **XIE M** ; **LE K** ; **XU M** ; **HUANG C**. The potential of Diosgenin in treating psoriasis: Studies from HaCaT keratinocytes and imiquimod-induced murine model. *Life Sci.*, 15 January 2020, vol. 241, 117115